Europäisches Patentamt

European Patent Office

Office européen des brevets

(11) Publication number: **0 141 104**
**A1**

## (12) EUROPEAN PATENT APPLICATION

(21) Application number: 84109935.1

(22) Date of filing: 21.08.84

(51) Int. Cl.⁴: **C 12 M 1/24**

(30) Priority: 28.09.83 US 536935

(43) Date of publication of application:
15.05.85 Bulletin 85/20

(84) Designated Contracting States:
BE DE FR GB IT NL SE

(71) Applicant: Becton Dickinson and Company
Mack Centre Drive
Paramus New Jersey 07652(US)

(72) Inventor: Franchere, Larry A.
518 Holly Avenue
Oxnard California(US)

(74) Representative: Selting, Günther, Dipl.-Ing. et al,
Deichmannhaus am Hauptbahnhof
D-5000 Köln 1(DE)

(54) Culture Flask.

(57) A culture flask (20) comprises a top wall (24), a bottom wall (25) and a continuous side wall )26) joining the top wall and the bottom wall. A neck (35) originates at an intersecting portion (34) of the top wall and the side wall and projects outwardly therefrom at an angle directed away from the bottom wall. The neck has an opening (37) therethrough to provide access to the interior of the flask and is adapted to cooperate with a separate sealing member (41). A relief wall (38) extending between a portion of the bottom wall and the side wall defines a recess in the flask. This recess is large enough to allow stacking of individual flasks whereby the recess of the upper flask in such a stack provides clearance for the neck on the next lower flask in the stack so that the bottom wall of the upper flask freely contacts the top wall of the lower flask.

FIG.2

EP 0 141 104 A1

## CULTURE FLASK

## BACKGROUND OF THE INVENTION

1.  Field of the Invention.  The present invention relates to a culture flask and more particularly concerns a space efficient stackable culture flask for the growth of cells therein.

2.  Description of the Prior Art.  Culture flasks or bottles, containing growth media, are employed in the laboratory environment to grow cells and microorganisms.  Medicine bottles have been employed for this purpose.  When laid on its side, a medicine bottle provides a large interior surface area of culture medium for sampling via pipette, however, the capacity to hold liquid is limited to a depth which is the distance between the side wall of the bottle and the inside edge of the neck.

Rohde (U.S. Patent No. 3,449,210) teaches an improved culture bottle wherein the neck of the bottle is offset from the bottle center line so that, when the bottle is placed on its side, more liquid can be held without any liquid running out of the neck opening.

A further improvement in the field of culture bottles is taught by Froman et al. (U.S. Patent No. 3,870,602).  The Froman et al. culture bottle has an offset neck, like Rohde, and also, the neck is angled upwardly away from the side of the bottle which rests on the work surface.  The angled neck opening allows improved access for a pipette or other instrument into the interior of the bottle.  Also, the upwardly

-2-

angled neck appears to allow the bottle to hold more liquid than a bottle in which the neck is not angled. Froman et al. further teach that one wall of the bottle is a flat optically transparent plastic member which is ultrasonically sealed to the remainder of the body structure. The transparent surface allows the microscopic examination of the contents. However, the multipiece construction increases cost and may present assembly problems and offer potential for leakage.

In certain situations, it is desirable to grow cells in liquid medium while minimizing the growth of cells on the interior surface of the culture flask. For example, in growing hybridoma cells, which are sometimes grown for the antibodies they secrete during the growing process, it is known that the cells which are not anchored to the surface of the culture flask give off more antibody secretions. Here it is desirable to provide a culture flask made of a relatively non-adhering, non-protein binding material to minimize cell growth on the flask walls.

It is also desirable to have a flask made of material which is transparent for purpose of analysis. This flask should maximize interior volume and make efficient use of shelf and incubator space while still providing ready access to the culture media contained therein. Further, it is desirable to have one-piece construction in order to eliminate assembly problems, potential for leaks developing at the intersection of the components, and costs associated with multi-piece flask construction.

Culture flasks with offset and angled neck portions have been addressed by the prior art. However, there is still a need for a simple, straight-forward, easily fabricated culture flask which optimizes the use of shelf and incubator space while being stackable and still allowing easy access of a pipette or other instrument into the interior of the flask. There is also a need for a transparent, one-piece flask. Further, a flask made of relatively non-adhering, non-protein binding materials is desirable for specific applications, similar to those alluded to above.

## SUMMARY OF THE INVENTION

The culture flask of the present invention comprises a top wall, a bottom wall and a continuous side wall joining the top wall and the bottom wall. A neck originates at an intersecting portion of the top wall and the side wall and projects outwardly therefrom at an angle directed away from the bottom wall. The neck has an opening therethrough to provide access to the interior of the flask and is adapted to cooperate with a separate sealing member. A relief wall extending between a portion of the bottom wall and the side wall defines a recess in the flask. This recess is large enough to allow stacking of individual flasks wherein the recess of the upper flask in the stack provides clearance for the neck on the next lower flask so that the bottom wall of the upper flask freely contacts the top wall of the next lower flask.

-4-

In accordance with the preferred embodiment of the present invention, a culture flask assembly comprises a rigid one-piece transparent flask having a substantially planar top wall and a substantially planar bottom wall positioned in substantially parallel relationship to the top wall. A side wall having four planar portions joins the top wall and the bottom wall. An angularly positioned upper relief wall intersects the top wall and at least two of the planar portions. A neck portion projects outwardly from the upper relief wall at an angle directed away from the bottom wall. This neck portion has a cylindrically shaped neck side wall defining an opening therethrough to provide access to the interior of the flask. The neck side wall has an exterior surface with a screw thread thereon. A cap is removably threaded onto the neck portion for closure of the opening. An angularly positioned lower relief wall intersects the bottom wall and at least two of the planar portions. This lower relief wall allows the stacking of individual flasks wherein the lower relief wall of the upper flask in the stack provides clearance for the cap on the lower flask so that the bottom wall of the upper flask freely contacts the top wall of the lower flask.

A number of advantages and objectives are attained consistent with the principles of the present invention. Primarily, the present invention provides a simple, straight-forward, easily fabricated culture flask which optimizes the use of shelf and incubator space while being stackable and

-5-

still allowing easy access of a pipette or other instrument into the interior of the flask. The present invention, in its preferred embodiment, also provides a one-piece flask made of transparent material which minimizes the potential for leaking while allowing observation of the contents therein.

## BRIEF DESCRIPTION OF THE DRAWINGS

Fig. 1 is a perspective view of the preferred culture flask assembly of the present invention;

Fig. 2 is a perspective view of the preferred culture flask assembly shown with the cap removed from the culture flask and the top wall partially cut-away to show part of the interior thereof;

Fig. 3 is a side corner elevation view of a stack of the preferred culture flask assemblies;

Fig. 4 is a front corner elevation view of the preferred culture flask assembly;

Fig. 5 is a side elevation view of the preferred culture flask assembly;

Fig. 6 is a cross-sectional view of the culture flask assembly of Fig. 5 taken along line 6-6;

Fig. 7 is a top elevation view of the preferred culture flask assembly showing volume measuring indicia on the top surface thereof; and

Fig. 8 is a side corner elevation view of an alternative embodiment of the present culture flask assembly.

## DETAILED DESCRIPTION

While this invention is satisfied by embodiments in many different forms, there is shown in the drawings and will herein be described in detail preferred embodiments of the invention with the understanding that the present disclosure is to be considered as exemplary of the principles of the invention and is not intended to limit the invention to the embodiments illustrated. The scope of the invention will be measured by the appended claims and their equivalents.

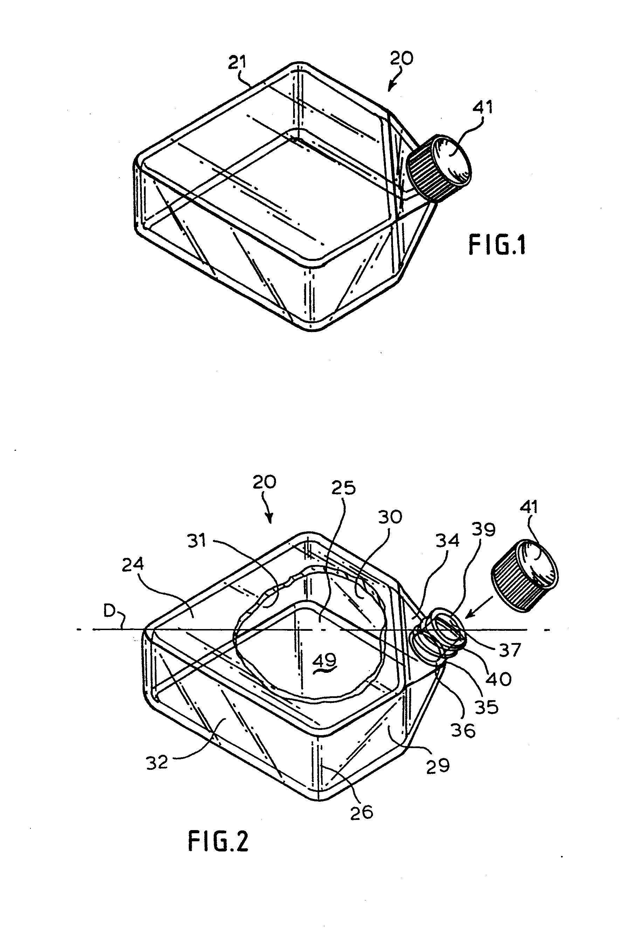

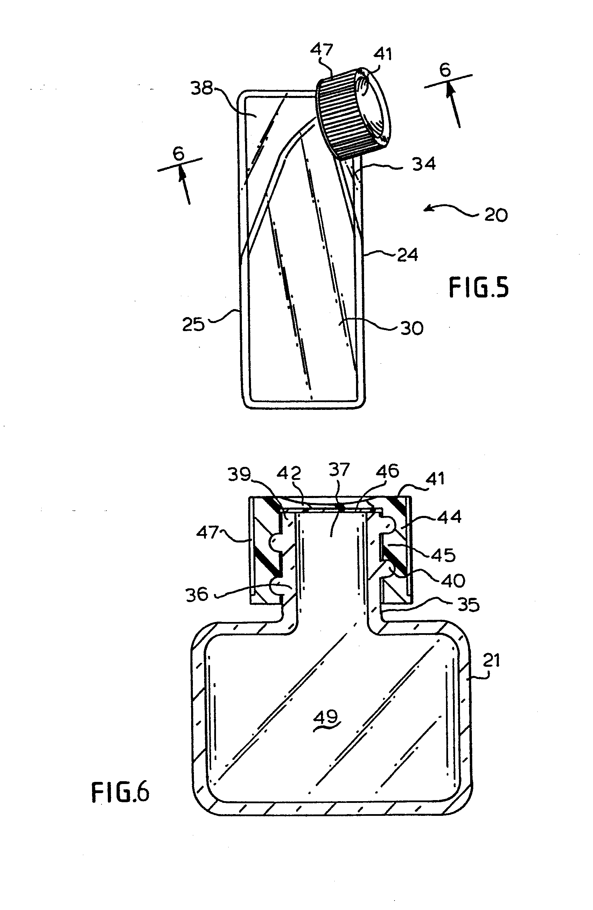

Adverting to Figs. 1 through 6, a preferred culture flask assembly 20 comprises a rigid one-piece transparent flask 21 and a screw cap 41. The culture flask includes a substantially planar top wall 24 and a substantially planar bottom wall 25 positioned in substantially parallel relationship to the top wall. A side wall 26 preferably having four planar portions 29, 30, 31 and 32, joins the top wall and the bottom wall. An angularly positioned upper relief wall 34, sloping downwardly from top wall 24, intersects the top wall and planar portions 29 and 30.

A neck portion 35 is integrally formed with and projects outwardly from the upper relief wall at an angle directed away from bottom wall 25. Neck portion 35 preferably includes a cylindrical neck side wall 36 defining an opening 37 and lip 39 at the open end of the neck. The angled neck, which originates at a height below the top wall, allows easy access of a pipette or other instrument into the interior of the flask and also allows for easier pouring since the liquid growth media, which may be in the flask, can be poured therefrom by tilting the flask at less of an angle than if the neck were positioned on the top wall. Also, it is preferred that the neck portion be positioned at a corner of the flask preferably in substantial alignment with diagonal line D, as best shown in Fig. 2. This diagonal alignment of the neck portion allows planar portions 31 and 32 to be positioned flush against the left or right side wall and the back wall of a storage container incubator to optimize the use of space available while still allowing ready access to the neck portion from the front of the storage container or incubator. Screw thread 40 is part of the neck side wall. Also provided on the flask is an angularly positioned lower relief wall 38, sloping upwardly from bottom wall 25, and intersecting the bottom wall and planar portions 29 and 30. The top wall, bottom wall, side wall planar portions, upper relief wall and lower relief wall preferably form an integral unit in the shape of a container having an interior volume 49 which is accessible only through opening 37 of the neck portion.

A cap 41 is provided to seal the neck opening. This cap preferably includes a top wall 42, a flexible liner 46 inside the cap adjacent to the top wall, and a circular side wall 44 with a screw thread 45 on the inside surface thereof. The flask is sealed by turning the cap until top wall 41 forces the flexible liner into firm contact with the lip 39 on the open end of the neck portion. Flexible liner 46 is provided to insure the complete sealing of the neck portion. Also provided is a knurled outer surface 47 on the exterior of the cap side wall to facilitate the tightening and removal of the cap by improving the user's grip thereon.

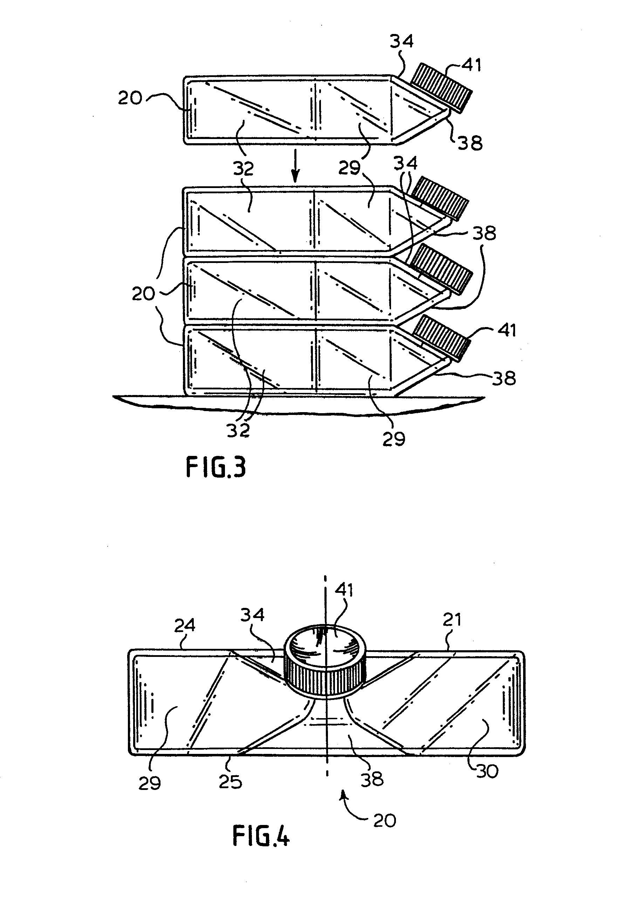

The combination of the angled position of neck 35 projecting from upper relief wall 34, and lower relief wall 38 allows stacking of individual culture flasks. As best shown in Fig. 3, the lower relief wall of the uppermost flask in the stack provides clearance for the neck, with cap 41 attached, of the next lower flask in the stack so that the bottom wall of the uppermost flask freely contacts the top wall of the next lower flask. In the preferred embodiment, the lower relief wall is at the same corner of the flask as the upper relief wall and the neck portion. This position is preferred because the caps of adjacent flasks in a stack are all positioned in a vertical line which allows easy access to all flasks. However, other positions for the lower relief wall are possible. For example, the lower relief wall can be positioned at the opposite corner of the flask from the upper relief wall wherein a stack of flasks of this construction would result in the caps of adjacent flasks being at opposite corners of the stack. Further, relief walls which run along an entire side of the flask, intersecting three side wall portions, although within the purview of this invention, are not preferred since they result in a greater reduction of the interior volume of the flask without a corresponding reduction in the shelf space taken up by the flask. Also, the preferred top wall and bottom wall have substantially flat exterior surfaces to provide a stable, large contact area between the flask and a work surface, and between adjacent flasks. However, it is within the purview of this invention to have other than completely flat top and bottom surfaces. For example, other complementary structure such as a raised portion on the top wall adapted to engage a recessed portion on the bottom wall will aid in maintaining the relative position of adjacent culture flasks in a stack. It can be seen that the culture flask and the stack of culture flasks illustrated and described herein have a straight sided, right angled geometric shape which optimizes the usage of shelf space and incubator space as compared to known prior art flasks.

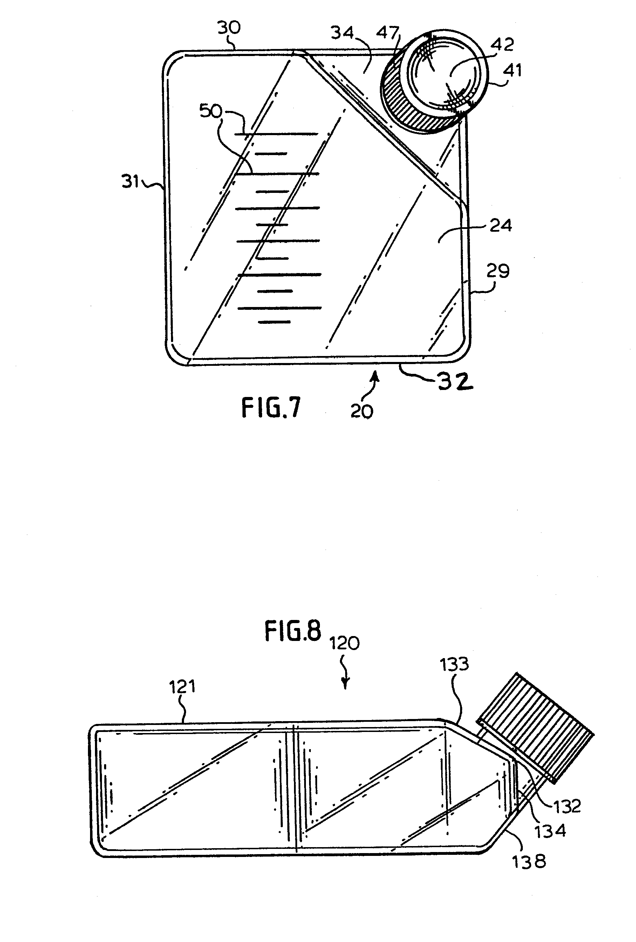

Referring now to Fig. 7, planar portions 31 or 32 of the side wall may be used as a stable surface for resting the culture flask on a work surface. When on the side postion the height of the liquid media that may be in the flask moves up or down a greater distance with each unit change of liquid volume within the culture flask than if the flask were resting on the bottom wall. Accordingly, top wall 24 or bottom wall 25, which is transparent in the preferred embodiment, become preferred surfaces for the inclusion of volume measuring indicia so that the volume of culture media in the culture flask can be measured without removing the media from the flask or measuring the media in a separate volume measuring device before adding it to the flask. The volume measuring indicia may be added to the exterior of the flask by hot stamping, silk screen painting, attaching a decal or sticker, or by any other suitable means. Also, the indicia may be formed by the process used to form the culture flask. Further, if the manufacturing process chosen to form the culture flask produces a raised parting line on planar portion 32, it may be desirable to provide feet or projections extending outwardly from this planar portion to provide a more stable base for the flask when resting on a work surface.

Turning now to Fig. 8, an alternative embodiment of the present culture flask assembly 120 includes culture flask 121 with upper relief wall 132 which consists of first portion 133 and a second portion 134 and lower relief wall 138. Figure 8 is thus an illustration that the upper relief wall and the lower relief wall may be made in various shapes in order to optimize the amount of culture media that can be held in the culture flask and/or to accomodate the manufacturing process. Accordingly, it is within the purview of the present invention to include a wide variety of shapes for the upper relief wall and the lower relief wall including, but not limited to, planar, multiplanar, curved and the like.

In use for growing cells, the user removes the cap from the culture flask assembly and adds the desired amount of liquid culture media. The volume of culture media in the flask can be determined by standing the flask on its side and observing the relative position between the indicia on the top wall of the flask and the height of the liquid in the flask. The flask is then returned to a position where its bottom wall is on the work surface. Then, the culture medium is inoculated with cells of the type to be grown, and the cap is firmly replaced onto the neck portion, sealing the flask. This process is repeated with additional flasks. The flasks are then stacked, one on top of another, and placed in an incubator for the time necessary to grow the desired number of cells. Since the preferred culture flask is transparent, the cells in the flasks may be periodically examined using an inverted microscope to look through the bottom wall of the flask.

Although a wide variety of rigid materials are suitable for the flask, transparent thermoplastic materials, with relatively non-adhering, non-protein binding properties, which are amenable to a blow molding process are preferred. Some of these materials are ABS, polystyrene and polymethylmethacrylate. A wide variety of rigid materials are suitable for the cap, however, thermoplastic materials such as polypropylene and polyethylene are preferred. Thermoplastic material such as polytetrafluoroethylene is preferred for the flexible liner. It is preferred that all elements of the culture flask should be sterile when used. Accordingly, materials should be selected for compatability with the sterilization process being used.

Thus, it can be seen that the present invention provides a simple, straight-forward, easily fabricated culture flask which optimizes the use of shelf and incubator space while being stackable and still allowing easy access of a pipette or other instrument into the interior of the flask. The preferred embodiment of the present invention also provides a one-piece construction which minimizes the potential for leaking and a transparent structure which allows observation and evaluation of the contents therein. The preferred embodiment provides volume measuring indicia and may be made of material with non-adhering, non-protein binding properties.

WHAT IS CLAIMED IS:

1. A culture flask comprising:

a top wall;

a bottom wall in substantially parallel relationship with said top wall;

a continuous side wall joining said top wall and said bottom wall;

an angularly positioned upper relief wall intersecting said top wall and said side wall;

a neck portion projecting outwardly from said upper relief wall at an angle directed away from said bottom wall, said neck portion having an opening therethrough to provide access to the interior of said flask;

engagement means for allowing said neck portion to cooperate with a separate sealing member; and

an angularly positioned lower relief wall intersecting said bottom wall and said side wall, said lower relief wall allowing the stacking of individual flasks whereby the lower relief wall of the upper flask in such a stack provides clearance for the neck portion on the lower flask in the stack so that the bottom wall of the upper flask freely contacts the top wall of the lower flask.

2. A culture flask comprising:

a top wall, a bottom wall and a continuous side wall joining said top wall and said bottom wall;

a neck originating at an intersecting portion of said top wall and said side wall and projecting outwardly therefrom at an angle directed away from said bottom wall, said neck having an opening therethrough
to provide access to the interior of said flask and
having means for cooperating with a separate sealing
member for closure of said opening; and

a relief wall extending between a portion of said
bottom wall and said side wall defining a recess therein, said recess being large enough to allow stacking of
individual flasks whereby the recess of the upper flask
in such a stack provides clearance for the neck on the
lower flask in the stack so that the bottom wall of the
upper flask freely contacts the top wall of the lower
flask.

3. A culture flask assembly comprising:

a rigid one-piece transparent flask having a substantially planar top wall and a substantially planar
bottom wall positioned in substantially parallel re-
lationsship to said top wall;

a side wall having four planar portions joining said
top wall and said bottom wall;

an angularly positioned upper relief wall intersecting said top wall and at least two of said planar
portions;

a neck portion projecting outwardly from said upper
relief wall at an angle directed away from said bottom
wall, said neck portion having a cylindrically shaped
neck side wall defining an opening therethrough to provide access to the interior of said flask, said neck
side wall having an exterior surface with a screw
thread thereon;

a cap removably threaded onto said neck portion for
closure of said opening;

an angularly positioned lower relief wall intersecting said bottom wall and at least two of said planar portions, said lower relief wall allowing the stacking of individual flasks whereby the lower relief wall of the upper flask in such a stack provides clearance for the cap on the lower flask in the stack so that the bottom wall of the upper flask freely contacts the top wall of the lower flask.

4. The culture flask of one of claims 2 to 3 wherein said bottom wall includes a substantially flat exterior bottom surface.

5. The culture flask of one of claims 1 to 4 wherein said top wall includes a substantially flat exterior top surface.

6. The culture flask of one of claims 1 to 5 wherein said side wall includes four planar portions positioned around the periphery of said flask.

7. The culture flask of claim 6 wherein said planar portions are positioned in substantially parallel pairs.

8. The culture flask of claim 6 wherein said neck portion is positioned at a corner of said flask and is diagonally aligned therewith.

9. The culture flask of one of claims 1 to 8 wherein said neck portion includes a neck side wall having an interior and an exterior surface, said exterior surface being cylindrically shaped.

10. The culture flask of claim 9 wherein said engaging means includes a screw thread on said exterior surface of said neck side wall, said thread being adapted to mate with a separate threaded sealing member.

11. The culture flask of one of claims 1 to 10 further made of a one-piece construction.

12. The culture flask of one of claims 1 to 11 wherein said upper relief wall has a substantially planar shape.

13. The culture flask of one of claims 1 to 12 wherein said lower relief wall has a substantially planar shape.

14. The culture flask of one of claims 1 to 13 wherein said top wall further includes volume measuring indicia oriented with one of said planar portions.

15. The culture flask of one of claims 1 to 14 wherein said flask is made of transparent material.

16. The culture flask of claim 15 wherein said transparent material is a thermoplastic material.

17. The culture flask of claim 16 wherein said thermoplastic material is selected from the group consisting of ABS, polystyrene and polymethyl-methacrylate.

18. A stack of culture flasks comprising a plurality of the culture flasks of one of claims 2 to 17 in which the bottom wall and the top wall of adjacent flasks are in contact with each other.

0141104

FIG.1

FIG.2

0141104

FIG.3

FIG.4

**FIG.5**

**FIG.6**

FIG.7

FIG.8

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | US-A-2 920 777 (H.G. COLE)<br><br>* figures; claims * | 1-7,9-13,15-18 | C 12 M 1/24 |
| Y | | 8,14 | |
| X | GB-A- 25 912 (M. ILLING) (A.D. 1913)<br>* figures * | 1-5,9,11 | |
| Y | | 8 | |
| A | US-A-2 947 116 (W.R. EARLE et al.)<br>* figures 1,2 * | 8 | |
| X | CH-A- 274 793 (N.V. MAATSCHAPPIJ TOT EXPLOITATIE DER OLIEFABRIEKEN CALVE-DELFT)<br>* figures * | 1-3,6,7,9-11,15-17 | **TECHNICAL FIELDS SEARCHED (Int. Cl.4)**<br><br>C 12 M<br>B 01 L<br>B 43 L<br>B 65 D |
| Y | | 14 | |
| D,A | US-A-3 870 602 (SEYMOUR FROMAN et al.) | | |
| D,A | US-A-3 449 210 (P.A. ROHDE et al.)<br><br>--- -/- | | |

The present search report has been drawn up for all claims

| Place of search | Date of completion of the search | Examiner |
|---|---|---|
| THE HAGUE | 30-01-1985 | COUCKE A.O.M. |

# EUROPEAN SEARCH REPORT

European Patent
Office

| DOCUMENTS CONSIDERED TO BE RELEVANT | | | Page 2 | |
|---|---|---|---|---|
| Category | Citation of document with indication, where appropriate, of relevant passages | Relevant to claim | CLASSIFICATION OF THE APPLICATION (Int. Cl.4) | |
| A | US-A-4 334 028 (J.L. CARVER)<br>* figures *<br><br>---- | 3 | | |
| | | | TECHNICAL FIELDS SEARCHED (Int. Cl.4) | |

The present search report has been drawn up for all claims

| Place of search | Date of completion of the search | Examiner |
|---|---|---|
| THE HAGUE | 30-01-1985 | COUCKE A.O.M. |